(19) Europäisches Patentamt
European Patent Office
Office européen des brevets

(11) **EP 2 455 740 B1**

(12) **FASCICULE DE BREVET EUROPEEN**

(45) Date de publication et mention
de la délivrance du brevet:
**04.09.2013 Bulletin 2013/36**

(51) Int Cl.:
***G01N 3/32*** *(2006.01)*

(21) Numéro de dépôt: **11189388.9**

(22) Date de dépôt: **16.11.2011**

(54) **Procédé et dispositif de détermination non destructive du module de rupture d'une pièce en bois**

Verfahren und Vorrichtung zur nicht-destruktiven Bestimmung des Reißmoduls eines Holzstücks

Method and device for non-destructive determination of the modulus of rupture of a wooden part

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.11.2010 FR 1059437**

(43) Date de publication de la demande:
**23.05.2012 Bulletin 2012/21**

(73) Titulaire: **Innodura**
**69100 Villeurbanne (FR)**

(72) Inventeurs:
• **Robin, Maxime**
**69300 Caluire et Cuire (FR)**
• **Beguet, Bernard**
**69210 Saint Bel (FR)**

(74) Mandataire: **Maureau, Philippe et al**
**Cabinet Germain & Maureau**
**B.P. 6153**
**69466 Lyon Cedex 06 (FR)**

(56) Documents cités:
**WO-A1-88/10415      WO-A1-2009/014463**
**GB-A- 1 244 699      US-A- 3 513 690**

## Description

La présente invention concerne un procédé et un dispositif de détermination non destructive du module de rupture d'une pièce en bois.

Il est courant d'utiliser des pièces en bois comme éléments porteurs et résistants d'une construction ou d'une structure. Aussi, il est nécessaire d'assurer un classement satisfaisant des pièces en bois en fonction de leur résistance à la rupture de manière à éviter une utilisation inappropriée de pièces en bois de faibles résistances mécaniques par exemple pour la réalisation de structures porteuses.

Afin d'assurer un tel classement, il est connu d'estimer de manière non destructive le module de rupture de chaque pièce en bois à partir d'un calcul du module d'élasticité de cette dernière.

Un premier dispositif de détermination du module de rupture d'une pièce en bois connu comprend des moyens d'excitation agencés pour exciter une première extrémité d'une pièce en bois, des moyens de détection agencés pour détecter, au niveau de l'autre extrémité de la pièce en bois, la réponse acoustique ou vibratoire de ladite pièce en bois en réponse à l'excitation, des moyens de calcul agencés pour calculer le module d'Young longitudinal de la pièce en bois à partir de la mesure de la première fréquence de résonance du spectre fréquentiel de la réponse détectée par les moyens de détection, et des moyens d'estimation du module de rupture à partir du module d'Young calculé.

Un tel dispositif de détermination présente l'inconvénient de n'être implantable que dans des lignes de production comportant des moyens de convoyage agencés pour déplacer les pièces en bois perpendiculairement à leur direction longitudinale, de manière à laisser un accès aux extrémités de chaque pièce de bois.

En outre, une estimation du module de rupture uniquement à partir du module d'Young longitudinal ne tient pas compte de l'anisotropie du bois, ce qui engendre des erreurs importantes de classement des pièces en bois.

De plus, étant donné que la présence et l'importance d'éventuels défauts dans une pièce en bois n'ont qu'une faible influence sur les premières fréquences de résonance du spectre fréquentiel de la réponse détectée par les moyens de détection, une estimation du module de rupture à partir du module d'Young calculé uniquement en fonction de la mesure de la première fréquence de résonance ne tient pas suffisamment compte de la présence et de l'importance d'éventuels défauts dans la pièce en bois.

Or, il est connu que la résistance à la rupture d'une pièce en bois varie considérablement en fonction de la présence ou non de noeuds ou d'autres défauts dans ladite pièce en bois. Il en résulte ainsi une estimation faussée du module de rupture.

Par conséquent, le dispositif de détermination précédemment décrit ne permet pas un classement satisfaisant des pièces en bois en fonction de leur résistance à la rupture.

Le document EP 1 950 561 décrit un dispositif de contrôle de la résistance mécanique d'une pièce en bois dans une ligne de production, comportant notamment une caméra agencée pour détecter localement d'éventuel défaut dans la pièce en bois, des moyens de calcul de la densité de la pièce en bois, et un couple excitateur-récepteur agencés pour permettre la détermination du module d'élasticité longitudinal de ladite pièce en bois, le module d'élasticité étant calculé à partir de l'analyse de la première fréquence de résonnance de compression.

Bien que le dispositif de contrôle décrit dans le document EP 1 950 561 améliore la précision de l'estimation du module de rupture du fait de l'utilisation d'une caméra, il conserve une hypothèse fausse d'isotropie du bois qui engendre des erreurs importantes de classement des pièces en bois.

En outre, la caméra peut fournir des informations erronées quant à la surface de la pièce en bois compte tenu de l'atmosphère poussiéreuse régnant dans une ligne de production de pièces en bois, ce qui augmente encore les erreurs de classement des pièces en bois.

Le document CA 2 497 199 décrit un procédé adapté pour le calcul du module d'élasticité d'une pièce en bois. Le procédé comporte plus particulièrement les étapes consistant à envoyer une impulsion ultrasonore à travers la pièce en bois, à mesurer la vitesse de transmission de l'impulsion ultrasonore à travers le bois, et à déterminer le module d'élasticité de la pièce en bois en fonction de la vitesse de transmission de l'impulsion ultrasonore à travers le bois.

Au-delà des considérations scientifiques non prises en compte (à savoir l'anisotropie du bois), cette technique de détermination du module d'élasticité se révèle peu fiable car fortement conditionnée par le contact boisexcitateur/récepteur. De plus, cette technique n'est pas adaptée pour être mise en oeuvre dans une ligne de production puisqu'elle nécessite l'arrêt des pièces de bois pour permettre le contact de ces dernières avec le couple excitateur-récepteur.

Les dispositifs décrits précédemment ne permettent donc pas d'assurer un classement satisfaisant des pièces en bois en fonction de leur résistance mécanique, et nécessitent fréquemment un déclassement des pièces en bois afin d'éviter tout accident lié à une utilisation inappropriée de ces dernières, ce qui entraîne une dévalorisation de la production.

Un procédé plus élaboré de détermination du module d'élasticité et du module de rupture utilisant une combinaison de capteurs de vision, à rayons X et dimensionnel est décrit dans le document WO 2004106918. Le procédé décrit dans ce document est basé sur une description locale de la fibre de bois et de ses défauts. Le procédé intègre les informations précédentes dans un modèle de la pièce en bois pour la détermination des modules d'élasticité et de rupture. Cette technique, bien que précise, se révèle difficilement généralisable en ligne de production, compte tenu notamment des coûts élevés des systèmes à rayon X et des risques d'irradiation du personnel mettant en oeuvre cette technique.

Le document WO 88/10415 divulgue les caractéristiques du préambule des revendications 1 et 12.

La présente invention vise à remédier à ces inconvénients.

Le problème technique à la base de l'invention consiste donc à fournir un procédé et un dispositif de détermination non destructive du module de rupture d'une pièce en bois qui soit fiable, simple et économique, qui puisse s'incorporer aisément dans une ligne de production, tout en permettant d'opérer à grande vitesse

A cet effet, la présente invention concerne un procédé de détermination non destructive du module de rupture d'une pièce en bois selon la revendication 1.

L'analyse d'une première bande de fréquences, dite basse fréquence, d'au moins l'une des réponses détectées permet d'identifier des comportements vibratoires globaux de la pièce en bois, et donc, en combinant ces résultats avec la mesure de la densité de la pièce en bois, d'évaluer les propriétés mécaniques de la fibre (modules d'élasticité, cisaillement...) de cette dernière à partir de modèle de calcul connu de l'homme du métier.

L'analyse d'une bande de fréquences, dite haute fréquence, de chaque réponse détectée permet d'identifier des comportements vibratoires locaux de courtes longueurs d'onde dépendants des propriétés mécaniques locales de la pièce en bois et de la présence ou non de défauts dans cette dernière, et donc de déterminer, pour chaque zone excitée de la pièce en bois, un critère local représentatif de la présence ou non de défaut et de la qualité de la fibre du bois.

Le fait de déterminer le module de rupture de ladite pièce en bois directement ou indirectement en fonction du ou des critères locaux déterminés à l'étape h) permet de tenir compte au moins en partie des propriétés mécaniques locales de la pièce en bois, et notamment de la présence et de l'importance d'éventuels défauts dans la pièce en bois, ce qui assure une estimation améliorée du module de rupture, et donc un classement mécanique améliorée de ladite pièce en bois.

De façon préférentielle, l'étape a) consiste à exciter transversalement au moins une zone prédéterminée de la pièce en bois. Ainsi, le procédé selon l'invention est aussi bien adapté à un déplacement longitudinal de la pièce de bois dans la zone d'excitation et de détection, qu'à un déplacement transversal de ladite pièce en bois. Le procédé selon l'invention est donc adapté pour être mis en oeuvre dans tout type de ligne de production.

De préférence, l'étape a) consiste à exciter au moins une zone centrale de la pièce en bois. Le fait d'exciter au moins la zone centrale de la pièce en bois permet de déterminer le module de rupture en tenant compte des propriétés mécaniques locales de la zone centrale de la pièce en bois, c'est-à-dire de la zone de la pièce en bois la plus sensible à la rupture. Ces dispositions permettent ainsi d'améliorer encore l'estimation du module de rupture, et donc le classement de la pièce en bois.

Avantageusement, l'étape a) consiste à exciter transversalement plusieurs zones prédéterminées de la pièce en bois décalées longitudinalement.

En répétant l'opération excitation-détection-analyse le long de la pièce en bois et en intégrant les différents critères locaux dans l'étape de détermination du module de rupture et/ou de la propriété mécanique de la fibre à partir de laquelle le module de rupture est déterminé, le procédé selon l'invention permet une détermination du module de rupture en tenant compte des propriétés mécaniques locales de la pièce en bois, et donc de l'anisotropie du bois et de la présence ou non de défauts dans ladite pièce en bois. Il en résulte une estimation encore plus précise du module de rupture, et de ce fait un classement mécanique encore plus précis de ladite pièce en bois.

Selon un mode de mise en oeuvre, le procédé comprend une étape consistant à déplacer longitudinalement la pièce en bois entre chaque étape d'excitation d'une zone prédéterminée de la pièce en bois.

De préférence, l'au moins une propriété mécanique de la fibre de la pièce en bois à partir de laquelle est déterminé le module de rupture est le module d'Young.

Selon un mode de mise en oeuvre du procédé, l'étape g) comprend une étape consistant à comparer la deuxième bande de fréquences du spectre fréquentiel de chaque réponse vibratoire et/ou acoustique détectée avec des deuxièmes bandes de fréquences de spectres fréquentiels de réponses vibratoires et/ou acoustiques de référence. Les réponses vibratoires et/ou acoustiques de référence sont par exemple obtenues à partir d'un modèle éléments finis de référence et/ou de résultats expérimentaux sur des pièces étalonnées, voir sur la même pièce de bois. La réponse haute fréquence d'une zone de la pièce en bois comportant des défauts diffère (en fréquence et en niveau) de la réponse d'une zone de la pièce en bois sans défaut. De même, la réponse haute fréquence d'une zone de la pièce en bois comportant peu de défauts diffère (en fréquence et en niveau) de la réponse d'une zone de la pièce en bois comportant beaucoup de défauts. Ainsi, une telle étape de comparaison permet de déterminer aisément à l'étape h) différents critères en fonction de la présence et de l'importance des défauts dans la pièce en bois.

Par exemple, l'étape g) comprend une étape consistant à calculer au moins une caractéristique représentative (telle que le niveau) de la deuxième bande de fréquences du spectre fréquentiel de chaque réponse vibratoire et/ou acoustique détectée, et à comparer lesdites caractéristiques calculées aux valeurs correspondantes des deuxièmes bandes de fréquences des spectres fréquentiels des réponses vibratoires et/ou acoustiques de référence.

Selon un autre mode de mise en oeuvre du procédé, l'étape g) comprend une étape consistant à calculer au moins une caractéristique représentative de la deuxième bande de fréquences du spectre fréquentiel de chaque réponse vibratoire et/ou acoustique détectée, telle que par exemple :

- le niveau de la deuxième bande de fréquences du spectre fréquentiel de chaque réponse vibratoire et/ou acoustique détectée,
- la fréquence de résonance d'au moins une émergence tonale appartenant à la deuxième bande spectrale du spectre fréquentiel de chaque réponse vibratoire et/ou acoustique détectée,
- la valeur d'émergence d'au moins une émergence tonale appartenant à la deuxième bande spectrale du spectre fréquentiel de chaque réponse vibratoire et/ou acoustique détectée, et/ou
- le rapport entre le niveau d'au moins une émergence tonale appartenant à la deuxième bande spectrale du spectre fréquentiel de chaque réponse vibratoire et/ou acoustique détectée et le niveau d'une bande critique centrée sur la fréquence de résonance de ladite émergence tonale en enlevant l'apport de ladite émergence tonale.

Un tel rapport est communément appelé rapport de tonalité, rapport TNR ou « Tone-to-Noise Ratio », et la bande critique représente généralement +/- 10% de la fréquence de résonance de l'émergence correspondante.

De façon préférentielle, l'étape g) comprend une étape consistant à calculer la fréquence de résonance, la valeur d'émergence et/ou le rapport TNR de la plus importante émergence tonale appartenant à la deuxième bande spectrale du spectre fréquentiel de chaque réponse vibratoire et/ou acoustique détectée.

Selon ce mode de mise en oeuvre, l'étape h) consiste à déterminer chaque critère relatif à chaque zone excitée de la pièce en bois à partir de l'au moins une caractéristique représentative correspondante calculée à l'étape g) et d'abaques de calcul. Les abaques de calcul sont de préférence déterminés de manière empirique, c'est-à-dire à partir de tests effectués sur des pièces en bois étalons.

Avantageusement, le procédé comprend une étape préalable aux étapes d'analyse et consistant à effectuer une transformée spectrale, de préférence de type FFT, de chaque réponse vibratoire et/ou acoustique détectée.

De façon avantageuse, l'étape d) comprend une étape consistant à mesurer les fréquences de résonance de plusieurs modes de résonance appartenant à la première bande de fréquences analysée.

De façon préférentielle, l'étape e) et/ou l'étape f) comprend une étape consistant à pondérer l'impact de chaque critère dans la détermination de l'au moins une propriété mécanique de la fibre et/ou du module de rupture en fonction de la localisation de la zone excitée correspondante.

Selon un mode de mise en oeuvre du procédé, l'étape f) consiste à déterminer le module de rupture par une analyse multiparamétrique telle que $MoR=F(MoE, \rho, C_N)$.

Selon un autre mode de mise en oeuvre du procédé, l'étape e) comprend les étapes consistant à :

- calculer au moins une valeur de module d'Young à partir de la densité de la pièce en bois déterminée à l'étape c) et de l'analyse effectuée à l'étape d),
- corriger l'au moins une valeur de module d'Young à l'aide des critères déterminés lors de l'étape h),
- déterminer le module de rupture de la pièce en bois à partir de l'au moins une valeur de module d'Young corrigée.

De préférence, l'étape a) consiste à impacter la pièce en bois à l'aide d'un dispositif d'impaction.

Avantageusement, le procédé de détermination comprend en outre les étapes consistant à établir un classement de la pièce en bois en fonction du module de rupture déterminé à l'étape f), et à trier la pièce en bois en fonction de l'étape de classement.

Selon un mode de mise en oeuvre, le procédé de détermination comprend une étape consistant à découper au moins une partie d'extrémité de la pièce en bois si cette partie présente une qualité inférieure au reste de la pièce en bois.

Selon un mode de mise en oeuvre, la première bande de fréquences est comprise entre 0 et 1000 Hz et la deuxième bande de fréquence est comprise entre 1000 Hz et 5000 Hz.

La présente invention concerne également un dispositif de détermination non destructive du module de rupture d'une pièce en bois, selon la revendication 12.

Avantageusement, les moyens d'excitation sont agencés pour exciter transversalement au moins une zone prédéterminée de la pièce en bois.

De préférence, le dispositif de détermination comprend en outre des moyens de déplacement agencés pour déplacer longitudinalement la pièce en bois relativement aux moyens d'excitation et de détection, les moyens de déplacement comportant de préférence des moyens de convoyage agencés pour convoyer la pièce en bois le long des moyens d'excitation et de détection.

Avantageusement, les moyens d'excitation comportent des moyens d'impaction.

De préférence, les moyens de détection sont agencés pour détecter au moins le champ vibratoire, l'intensité acoustique et/ou la pression acoustique de la réponse de la pièce en bois.

Selon un mode de réalisation, les moyens de détection comportent au moins un microphone, et avantageusement une pluralité de microphones.

Selon un autre mode de réalisation, les moyens de détection comportent au moins un vibromètre laser ou un capteur laser de déplacement.

L'utilisation d'un réseau de microphones, d'un vibromètre laser ou d'un capteur laser de déplacement permet de mesurer ou de calculer le champ vibro-acoustique directement à la surface de la pièce en bois, ce qui à pour avantage d'augmenter considérablement le rapport signal sur bruit de cette mesure in situe sans contact. Le réseau de microphones présente le dernier avantage de filtrer le champ évanescent de la réponse et d'en conserver uniquement la partie prorogative qui est intrinsèquement locale.

De toute façon l'invention sera bien comprise à l'aide de la description qui suit en référence au dessin schématique annexé représentant, à titre d'exemple non limitatif, une forme d'exécution de ce dispositif de détermination.

Figure 1 est une vue partielle en perspective d'un dispositif de détermination selon l'invention.
Figure 2 es une vue schématique de côté du dispositif de détermination de la figure 1.
Figure 3 est un diagramme schématisant la détermination du module de rupture.
Figure 4 est un diagramme représentant le spectre fréquentiel d'une réponse acoustique et/ou vibratoire détectée.
Figure 5 est un diagramme représentant deux spectres fréquentiels superposés correspondant respectivement à une zone de la pièce en bois comportant des défauts et à une zone de la pièce en bois dépourvue de défaut.
Figure 6 est une vue schématique de dessus d'un dispositif de détermination selon l'invention selon une variante de réalisation.
Figure 7 est un diagramme représentant la variation du critère relatif à la présence et à l'importance d'un éventuel défaut dans un zone prédéterminée de la pièce en bois en fonction du rapport TNR calculé par les deuxièmes moyens d'analyse.

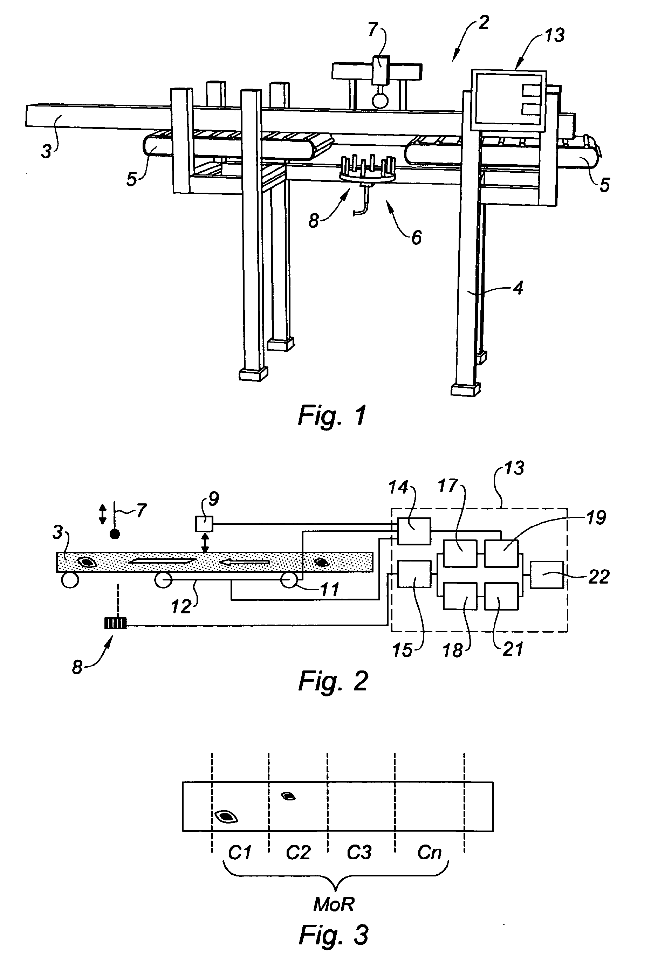

La figure 1 représente un dispositif de détermination non destructive 2 du module de rupture d'une pièce en bois 3. La pièce en bois peut être par exemple une planche ou une poutre.

Le dispositif de détermination 2 comprend un châssis 4 supportant des moyens de déplacement agencés pour déplacer longitudinalement ladite pièce en bois 3 à vitesse constante. Les moyens de déplacement comportent de façon avantageuse deux convoyeurs à bande 5 écartés l'un par rapport à l'autre de manière à délimiter une zone d'excitation et de détection 6.

Le dispositif de détermination 2 comprend en outre des moyens d'excitation disposés au niveau de la zone d'excitation et de détection 6 et agencés pour exciter transversalement et localement la pièce en bois 3 de manière à générer des vibrations mécaniques dans la pièce en bois. Les moyens d'excitation comportent avantageusement un impacteur 7 agencé pour impacter la pièce en bois.

Le dispositif de détermination 2 comprend également des moyens de détection agencés pour détecter une réponse vibratoire et/ou acoustique de la pièce en bois 3 en réponse à ladite excitation. Les moyens de détection sont de préférence disposés à proximité de la pièce en bois 3. Comme plus particulièrement montré sur la figure 1, les moyens de détection comportent une pluralité de microphones 8 disposés au niveau de la zone d'excitation et de détection 6 et en regard de l'impacteur 7. Les microphones 8 sont de préférence disposés de manière annulaire et sont par exemple régulièrement espacés.

Le dispositif de détermination 2 comprend des moyens de mesure agencés pour mesurer les dimensions de la pièce en bois 3. Les moyens de mesure comportent avantageusement un capteur dimensionnel 9, tel qu'un télémètre infrarouge ou laser, agencé pour mesurer la largeur et l'épaisseur de la pièce en bois, le capteur dimensionnel étant de préférence associé à un codeur incrémental 11 agencé pour mesurer la longueur de la pièce en poids lors de son déplacement sur les convoyeurs 5.

Le dispositif de détermination 2 comprend de plus un système de pesée 12 relié aux convoyeurs et agencé pour mesurer la masse de la pièce en bois 3.

Le dispositif de détermination 2 comprend en outre une unité centrale de contrôle et de traitement de données 13.

Ladite unité centrale 13 comporte des moyens de détermination 14 de la densité de la pièce en bois reliés aux moyens de mesure 9, 11 et au système de pesée 12 et agencés pour calculer la densité de la pièce en bois à partir des mesures effectuées par les moyens de mesure et le système de pesée.

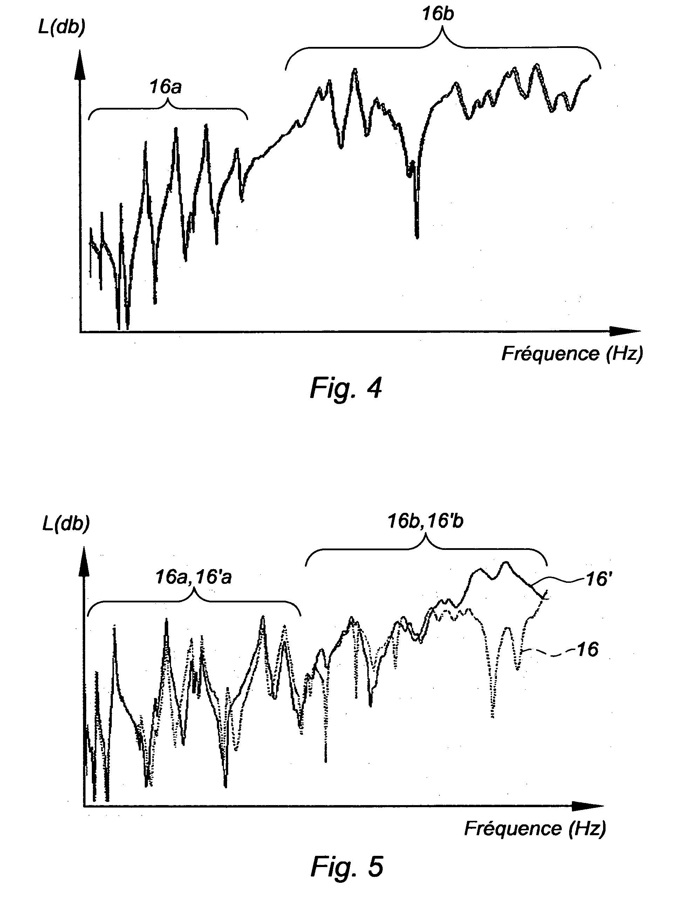

Ladite unité centrale 13 comporte des moyens de transformation 15 agencés pour effectuer une transformée spectrale, de préférence de type FFT, de chaque réponse vibratoire et/ou acoustique détectée. Le spectre fréquentiel 16 de l'une des réponses vibratoire et/ou acoustique détectée obtenu après transformation spectrale est représenté sur la figure 4. Le spectre fréquentiel 16 comporte une première bande de fréquence 16a, dite basse fréquence, comportant une pluralité de modes de flexion correspondant à la vibration de toute la pièce en bois et donc représentatifs des propriétés mécaniques de la fibre de la pièce en bois, et une deuxième bande de fréquences 16b, de fréquences supérieures à la première bande de fréquences 16a et dite haute fréquence, correspondant aux comportements vibratoires locaux de courtes longueurs d'onde dépendants des propriétés mécaniques locales de la pièce en bois et de la présence ou non de défauts dans cette dernière. On entend par défaut une imperfection de la pièce en bois, qui peut être par exemple un noeud ou une inclusion.

Comme montré sur la figure 5, le spectre fréquentiel 16 d'une réponse vibratoire et/ou acoustique détectée dans une zone de la pièce en bois comportant des défauts et le spectre fréquentiel 16' d'une réponse vibratoire et/ou acoustique détectée dans une zone de la pièce en bois dépourvue de défaut présentent des premières bandes de fréquences 16a, 16'a sensiblement identiques, mais des deuxièmes bandes de fréquences 16b, 16'b sensiblement différentes.

Ladite unité centrale 13 comporte en outre des premiers moyens d'analyse 17 agencés pour analyser la première bande de fréquences 16a du spectre fréquentiel d'au moins l'une des réponses vibratoires et/ou acoustiques détectées par les moyens de détection. Les premiers moyens d'analyse 17 sont plus particulièrement agencés pour mesurer les fréquences de résonance de plusieurs modes de flexion (par exemple de l'ensemble des modes de flexion) appartenant à chaque première bande de fréquences 16a analysée.

Ladite unité centrale 13 comporte également des deuxièmes moyens d'analyse 18 agencés pour analyser la deuxième bande de fréquences 16b du spectre fréquentiel de chaque réponse vibratoire et/ou acoustique détectée par les moyens de détection 8. Les deuxièmes moyens d'analyse 18 sont plus particulièrement agencés pour comparer la deuxième bande de fréquences 16b du spectre fréquentiel de chaque réponse vibratoire et/ou acoustique détectée avec des deuxièmes bandes de fréquences de spectres fréquentiels de réponses vibratoires et/ou acoustiques de référence.

Les deuxièmes moyens d'analyse 18 sont par exemple agencés pour calculer le niveau de la deuxième bande de fréquences 16b du spectre fréquentiel de chaque réponse vibratoire et/ou acoustique détectée, et à comparer lesdites valeurs calculées aux valeurs correspondantes des deuxièmes bandes de fréquences des spectres fréquentiels des réponses vibratoires et/ou acoustiques de référence.

Ladite unité centrale 13 comporte de plus :

- des moyens de détermination 19 agencés pour déterminer des propriétés mécaniques de la fibre de la pièce en bois, et notamment le module d'Young, à partir de la densité de la pièce en bois et de l'analyse effectuée par les premiers moyens d'analyse 17, c'est-à-dire de la mesure des fréquences de résonance des modes de flexion appartenant à chaque première bande de fréquences 16a analysée,
- des moyens de détermination 21 agencés pour déterminer, pour chaque zone excitée $Z_1$, $Z_2$, $Z_3$, ..., $Z_N$ de la pièce en bois, un critère $C_1$, $C_2$, $C_3$, ..., $C_N$ relatif à la présence et à l'importance d'un éventuel défaut dans ladite zone à partir de l'analyse effectuée par les deuxièmes moyens d'analyse 18, le critère pouvant par exemple est une valeur numérique variant d'une première valeur correspondant à une zone sans défaut à une deuxième valeur correspondant à une zone comportant de nombreux défauts,
- des moyens de détermination 22 agencés pour déterminer le module de rupture MoR de ladite pièce en bois à partir du module d'Young déterminé par les moyens de détermination 19 et en fonction des différents critères déterminés par les moyens de détermination 21.

Les moyens de détermination 19 sont plus particulièrement agencés d'une part pour calculer une valeur de module d'Young pour chaque fréquence de résonance mesurée par les premiers moyens d'analyse 17 à l'aide de la formule suivante : $MoE = \rho(\frac{2Lf_n}{n})^2$ dans laquelle MoE est le module de rupture, p est la densité de la pièce en bois, L est la longueur de la pièce en bois, n est un entier supérieur ou égale à 1 correspondant à un mode de flexion (1 correspondant au premier mode de flexion et n correspondant au énième mode de flexion) et $f_n$ est la fréquence de résonnance du mode de flexion correspondant, et d'autre part pour déterminer le module d'Young de la pièce en bois en moyennant les différentes valeurs de module d'Young calculées.

Les moyens de détermination 22 sont de préférence agencés pour pondérer l'impact de chaque critère dans la détermination du module de rupture en fonction de la localisation de la zone excitée correspondante.

Le procédé de détermination non destructive du module de rupture d'une pièce en bois à l'aide du dispositif de détermination préalablement décrit va maintenant être décrit.

Le procédé de détermination comprend les étapes suivantes consistant à :

- impacter transversalement au moins une zone prédéterminée de la pièce en bois 3 à l'aide de l'impacteur 7 de manière à générer des vibrations mécaniques dans la pièce en bois,
- détecter une réponse vibratoire et/ou acoustique de la pièce en bois en réponse à l'impaction de chaque zone prédéterminée à l'aide des moyens de détection 8,
- déplacer longitudinalement la pièce en bois 3 à l'aide des convoyeurs 5, et répéter les étapes d'impaction et de détection dans plusieurs zones prédéterminées de la pièce en bois décalées longitudinalement,
- mesurer les dimensions et la masse de la pièce en bois 3 à l'aide des moyens de mesure 9, 11 et du système de pesée 12, et déterminer sa densité à l'aide des moyens de détermination 14,
- effectuer une transformée spectrale, de préférence de type FFT, de chaque réponse vibratoire et/ou acoustique détectée à l'aide des moyens 15,
- analyser la première bande de fréquences 16a du spectre fréquentiel 16 d'au moins l'une des réponses vibratoires et/ou acoustiques détectées et mesurer les fréquences de résonance de plusieurs ou de l'ensemble des modes de flexion appartenant à ladite première bande de fréquences,
- déterminer des propriétés mécaniques de la fibre de la pièce en bois, telles que le module d'Young, à partir de la densité de la pièce en bois et des mesures des fréquences de résonance des modes de flexion de la première bande de fréquences analysée,

- analyser la deuxième bande de fréquences 16b du spectre fréquentiel 16 de chaque réponse vibratoire et/ou acoustique détectée, et comparer chaque deuxième bande de fréquences 16b analysée avec des deuxièmes bandes de fréquences de spectres fréquentiels de réponses vibratoires et/ou acoustiques de référence,
- déterminer, pour chaque zone excitée $Z_N$ de la pièce en bois, un critère $C_N$ relatif à la présence et à l'importance d'un éventuel défaut dans ladite zone à partir de la comparaison de chaque deuxième bande de fréquences analysée 16a avec des deuxièmes bandes de fréquences dites de référence,
- déterminer par une analyse multiparamétrique le module de rupture MoR de ladite pièce en bois 3 à partir du module d'Young déterminée à l'aide des moyens de détermination 19 et en fonction des différents critères déterminés à l'aide des moyens de détermination 21, le module de rupture MoR étant défini tel que MoR=F(MoE, $\rho$, $C_N$).

Avantageusement, l'étape d'analyse de chaque deuxième bande de fréquences 16a comprend une étape consistant à calculer le niveau de la deuxième bande de fréquences du spectre fréquentiel de chaque réponse vibratoire et/ou acoustique détectée, et l'étape de comparaison comprend une étape consistant à comparer lesdites valeurs calculées aux valeurs correspondantes des deuxièmes bandes de fréquences dites de référence.

De préférence, l'étape de détermination du module de rupture comprend une étape consistant à pondérer l'impact de chaque critère $C_N$ dans la détermination du module de rupture en fonction de la localisation de la zone excitée correspondante.

Selon une variante de réalisation, les moyens de détermination 19 sont agencés pour déterminer des propriétés mécaniques de la fibre de la pièce en bois, et notamment le module d'Young, à partir de la densité de la pièce en bois, de l'analyse effectuée par les premiers moyens d'analyse 17, et en fonction des différents critères déterminés par les moyens de détermination 21, tandis que les moyens de détermination 22 sont agencés pour déterminer le module de rupture de ladite pièce en bois à partir uniquement du module d'Young déterminé par les moyens de détermination 19.

Selon cette variante de réalisation, les deuxièmes moyens d'analyse 18 sont agencés pour calculer au moins une caractéristique représentative de la deuxième bande de fréquences du spectre fréquentiel de chaque réponse vibratoire et/ou acoustique détectée, telle que par exemple la fréquence de résonance, la valeur d'émergence et/ou le rapport TNR d'au moins une émergence tonale appartenant à la deuxième bande spectrale du spectre fréquentiel de chaque réponse vibratoire et/ou acoustique détectée.

De plus, les moyens de détermination 21 sont agencés pour déterminer chaque critère relatif à chaque zone excitée de la pièce en bois à partir de l'au moins une caractéristique représentative correspondante calculée par les deuxièmes moyens d'analyse 18 et d'abaques de calcul déterminés de manière empirique et préalablement mémorisés dans les moyens de détermination 21.

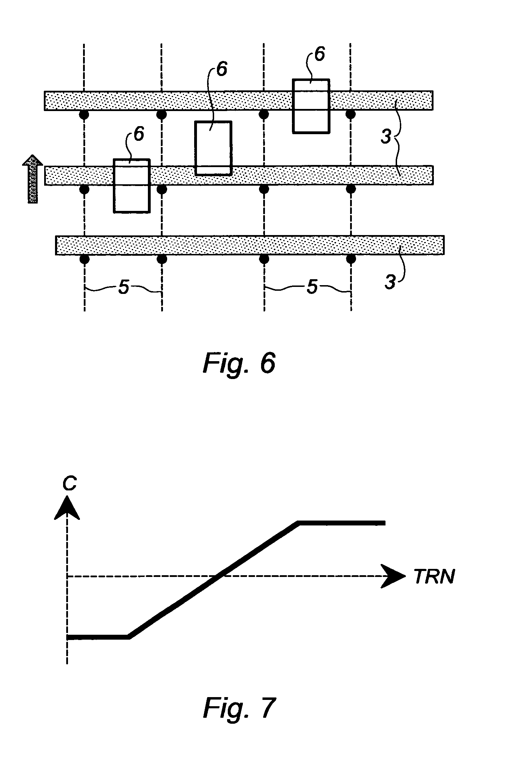

La figure 7 représente un exemple d'abaque de calcul représentant la variation du critère relatif à la présence et à l'importance d'un éventuel défaut dans une zone prédéterminée de la pièce en bois en fonction du rapport TNR calculé par les deuxièmes moyens d'analyse 18. Selon cet abaque de calcul, le critère est une valeur numérique exprimée en GPa et varie d'une première valeur minimale correspondant à une zone comportant de nombreux défauts à une deuxième valeur maximale correspondant à une zone sans défaut. Le critère varie d'une valeur par exemple de -1,5 GPa pour un rapport TNR inférieur à une première valeur prédéterminée à une valeur par exemple de +1,5 GPa pour un rapport TNR supérieur à une deuxième valeur prédéterminée, le critère variant de manière linéaire entre ces première et deuxième valeurs prédéterminées.

En outre, selon cette variante de réalisation, les moyens de détermination 19 sont agencés pour :

- calculer une valeur de module d'Young pour chaque fréquence de résonance mesurée par les premiers moyens d'analyse 17 à l'aide de la formule suivante :

$$MoE = \rho(\frac{2Lf_n}{n})^2$$ dans laquelle MoE est le module de rupture, p est la densité de la pièce en bois, L est la longueur de la pièce en bois, n est un entier supérieur ou égale à 1 correspondant à un mode de flexion (1 correspondant au premier mode de flexion et n correspondant au énième mode de flexion) et $f_n$ est la fréquence de résonnance du mode de flexion correspondant,
- définir un module d'Young local pour chaque zone excitée de la pièce en bois en moyennant les différentes valeurs de module d'Young calculées pour le spectre fréquentiel de la réponse vibratoire et/ou acoustique détectée correspondante,
- corriger chaque module d'Young local en y ajoutant le critère correspondant déterminés par les moyens de détermination 21,
- déterminer le module d'Young de la pièce en bois en fonction des différents modules d'Young locaux corrigés, et de préférence en pondérant l'impact de chaque module d'Young local corrigé en fonction de la localisation de la zone excitée correspondante.

Il doit être noté qu'il serait possible de prévoir des abaques de calcul consistant à faire varier le critère en fonction de la fréquence de résonnance et/ou de la valeur d'émergence de la plus importante émergence.

Selon une autre variante de réalisation, les moyens de détection pourraient comporter un vibromètre laser ou un capteur laser de déplacement en plus des microphones ou à la place de ces derniers.

Selon une variante de réalisation représentée sur la figure 6, le dispositif de détermination 2 comporte des convoyeurs 5 agencés pour déplacer les pièces en bois 3 perpendiculairement à leur direction longitudinale, et une pluralité de zones d'impaction et de détection 6 décalées les unes des autres et chacune pourvues de moyens d'impaction 7 et de détection 8. Selon cette variante de réalisation, les convoyeurs 5 comportent des chaînes de convoyage pourvues de taquets pousseurs destinés à déplacer les pièces en bois.

Selon un autre mode de mise en oeuvre du procédé selon l'invention, l'étape a) consiste à exciter longitudinalement une première extrémité d'une pièce en bois de manière à générer des vibrations mécaniques dans la pièce en bois, et l'étape b) consiste à détecter une réponse vibratoire et/ou acoustique de la pièce en bois en réponse à ladite excitation au niveau de l'autre extrémité de la pièce en bois. Selon ce mode de mise en oeuvre, il n'est pas possible de déterminer la position des éventuels défauts présents dans la pièce en bois, mais permet toutefois d'intégrer au moins en partie la présence de ces défauts dans la détermination du module de rupture de la pièce en bois.

Comme il va de soi, l'invention ne se limite pas à la seule forme d'exécution de ce dispositif de détermination, décrite ci-dessus à titre d'exemple, elle en embrasse au contraire toutes les variantes de réalisation.

**Revendications**

1.  Procédé de détermination non destructive du module de rupture d'une pièce en bois (3), comprenant les étapes consistant à :

    a) exciter au moins une zone prédéterminée de la pièce en bois de manière à générer des vibrations mécaniques dans la pièce en bois,
    b) détecter une réponse vibratoire et/ou acoustique de la pièce en bois en réponse à l'excitation de chaque zone prédéterminée,
    c) mesurer les dimensions et la masse de la pièce en bois de manière à déterminer sa densité,
    d) analyser une première bande de fréquences (16a) du spectre fréquentiel (16) d'au moins une réponse vibratoire et/ou acoustique détectée,
    e) déterminer au moins une propriété mécanique de la fibre de la pièce en bois à partir d'au moins la densité de la pièce en bois déterminée à l'étape c) et de l'analyse effectuée à l'étape d),
    f) déterminer le module de rupture (MoR) de ladite pièce en bois à partir d'au moins une propriété mécanique de la fibre de la pièce en bois déterminée à l'étape e),

    **caractérisé en ce qu'**il comprend en outre les étapes consistant à :
    g) analyser une deuxième bande de fréquences (16b), de fréquences supérieures à la première bande de fréquences, du spectre fréquentiel (16) de chaque réponse vibratoire et/ou acoustique détectée,
    h) déterminer, pour chaque zone excitée ($Z_N$) de la pièce en bois, un critère ($C_N$) relatif à la présence et à l'importance d'un éventuel défaut dans ladite zone à partir de l'analyse effectuée à l'étape g),

    le module de rupture et/ou l'au moins une propriété mécanique de la fibre de la pièce en bois à partir de laquelle est déterminé le module de rupture étant en outre déterminé en fonction du ou des critères déterminés à l'étape h).

2.  Procédé selon la revendication 1, dans lequel l'étape a) consiste à exciter transversalement au moins une zone prédéterminée de la pièce en bois.

3.  Procédé selon la revendication 2, dans lequel l'étape a) consiste à exciter transversalement plusieurs zones prédéterminées de la pièce en bois décalées longitudinalement.

4.  Procédé selon la revendication 3, lequel comprend une étape consistant à déplacer longitudinalement la pièce en bois entre chaque étape d'excitation d'une zone prédéterminée de la pièce en bois.

5.  Procédé selon l'une des revendications 1 à 4, dans lequel l'étape g) comprend une étape consistant à comparer la deuxième bande de fréquences du spectre fréquentiel de chaque réponse vibratoire et/ou acoustique détectée avec des deuxièmes bandes de fréquences de spectres fréquentiels de réponses vibratoires et/ou acoustiques de référence.

6.  Procédé selon l'une des revendications 1 à 5, dans lequel l'étape g) comprend une étape consistant à calculer au moins une caractéristique représentative de la deuxième bande de fréquences du spectre fréquentiel de chaque réponse vibratoire et/ou acoustique détectée.

7.  Procédé selon les revendications 5 et 6, dans lequel l'étape g) comprend une étape consistant à comparer lesdites caractéristiques représentatives calculées aux valeurs correspondantes des deuxièmes bandes de fréquences des spectres fréquentiels des réponses vibratoires et/ou acoustiques de référence.

8.  Procédé selon la revendication 6, dans lequel l'étape h) consiste à déterminer chaque critère relatif à chaque zone excitée de la pièce en bois à partir de l'au moins une caractéristique représentative correspondante calculée à l'étape g) et d'abaques de calcul.

9.  Procédé selon l'une des revendications 1 à 8, dans lequel l'étape d) comprend une étape consistant à mesurer les fréquences de résonance de plusieurs modes de résonnance appartenant à la première bande de fréquences analysée.

10.  Procédé selon l'une des revendications 1 à 9, dans lequel l'étape e) et/ou l'étape f) comprend une étape consistant à pondérer l'impact de chaque critère dans la détermination de l'au moins une propriété mécanique de la fibre et/ou du module de rupture en fonction de la localisation de la zone excitée correspondante.

11.  Procédé selon l'une des revendications 1 à 10, dans lequel l'étape a) consiste à impacter la pièce en bois à l'aide d'un dispositif d'impaction.

12.  Dispositif de détermination non destructive (2) du module de rupture d'une pièce en bois (3), comprenant :

- des moyens d'excitation (7) agencés pour exciter au moins une zone prédéterminée de la pièce en bois de manière à générer des vibrations mécaniques dans la pièce en bois,
- des moyens de détection (8) agencés pour détecter une réponse vibratoire et/ou acoustique de la pièce en bois en réponse à l'excitation de chaque zone prédéterminée,
- des moyens de mesure (9, 11, 12) agencés pour mesurer les dimensions et la masse de la pièce en bois, et des moyens de détermination (14) agencés pour déterminer la densité de la pièce en bois,
- des premiers moyens d'analyse (17) agencés pour analyser une première bande de fréquences (16a) du spectre fréquentiel (16) d'au moins une réponse vibratoire et/ou acoustique détectée,
- des premier moyens de détermination (19) agencés pour déterminer au moins une propriété mécanique de la fibre de la pièce en bois à partir d'au moins la densité de la pièce en bois et de l'analyse effectuée par les premiers moyens d'analyse, et
- des deuxièmes moyens de détermination (22) agencés pour déterminer le module de rupture de ladite pièce en bois à partir d'au moins une propriété mécanique de la fibre de la pièce en bois déterminée par les premiers moyens de détermination,
**caractérisé en ce qu'**il comprend en outre :
- des deuxièmes moyens d'analyse (18) agencés pour analyser une deuxième bande de fréquences (16b), de fréquences supérieures à la première bande de fréquences, du spectre fréquentiel (16) de chaque réponse vibratoire et/ou acoustique détectée,
- des troisièmes moyens de détermination (21) agencés pour déterminer, pour chaque zone excitée ($Z_N$) de la pièce en bois, un critère ($C_N$) relatif à la présence et à l'importance d'un éventuel défaut dans ladite zone à partir de l'analyse effectuée par les deuxièmes moyens d'analyse (18),

les premiers et/ou deuxièmes moyens de détermination (19, 22) étant en outre agencés pour déterminer le module de rupture et/ou l'au moins une propriété mécanique de la fibre de la pièce en bois à partir de laquelle est déterminé le module de rupture en fonction du ou des critères déterminés par les troisièmes moyens de détermination (21).

13.  Dispositif de détermination selon la revendication 12, dans lequel les moyens d'excitation (7) sont agencés pour exciter transversalement au moins une zone prédéterminée de la pièce en bois.

14.  Dispositif de détermination selon la revendication 12 ou 13, lequel comprend en outre des moyens de déplacement (5) agencés pour déplacer longitudinalement la pièce en bois relativement aux moyens d'excitation et de détection.

15.  Dispositif de détermination selon la revendication 14, dans lequel les moyens de déplacement comportent des moyens de convoyage agencés pour convoyer la pièce en bois le long des moyens d'excitation et de détection (7, 8).

**Patentansprüche**

1.  Verfahren zur zerstörungsfreien Bestimmung des Reißmoduls eines Holzstücks (3), das die folgenden Schritte umfasst:

a) Erregen mindestens einer vorbestimmten Zone des Holzstücks, um mechanische Vibrationen in dem Holzstück zu erzeugen,
b) Detektion einer Vibrations- und/oder akustischen Antwort des Holzstücks in Beantwortung auf die Erregung jeder vorbestimmten Zone,
c) Messen der Abmessungen und der Masse des Holzstücks, um seine Dichte zu bestimmen,
d) Analysieren eines ersten Frequenzbands (16a) des Frequenzspektrums (16) mindestens einer ermittelten Vibrations- und/oder akustischen Antwort,
e) Bestimmung mindestens einer mechanischen Eigenschaft der Faser des Holzstücks ausgehend von mindestens der in Schritt c) bestimmten Dichte des Holzstücks und der in Schritt d) durchgeführten Analyse,

f) Bestimmung des Reißmoduls (MoR) des Holzstücks ausgehend von mindestens einer in Schritt e) bestimmten mechanischen Eigenschaft der Faser des Holzstücks,

**dadurch gekennzeichnet dass** es ferner die Schritte umfasst, die darin bestehen:

g) Analysieren eines zweiten Frequenzbands (16b) mit höheren Frequenzen als das erste Frequenzband des Frequenzspektrums (16) jeder ermittelten Vibrations- und/oder akustischen Antwort,

h) Bestimmung für jede erregte Zone ($Z_N$) des Holzstücks eines Kriteriums ($C_N$), das sich auf die Anwesenheit und das Ausmaß eines eventuellen Fehlers in der Zone ausgehend von der in Schritt g) durchgeführten Analyse bezieht,

wobei das Reißmodul und/oder die mindestens eine mechanische Eigenschaft der Faser des Holzstücks, von der ausgehend das Reißmoduls bestimmt wird, ferner in Abhängigkeit von dem oder den in Schritt h) bestimmten Kriterium/Kriterien bestimmt wird.

2. Verfahren nach Anspruch 1, wobei Schritt a) darin besteht, mindestens eine vorbestimmte Zone des Holzstücks quer zu erregen.

3. Verfahren nach Anspruch 2, wobei Schritt a) darin besteht, mehrere, längs versetzte vorbestimmte Zonen des Holzstücks quer zu erregen.

4. Verfahren nach Anspruch 3, wobei es einen Schritt umfasst, der darin besteht, das Holzstück zwischen jedem Erregungsschritt einer vorbestimmten Zone des Holzstücks längs zu verlagern.

5. Verfahren nach einem der Ansprüche 1 bis 4, wobei der Schritt g) einen Schritt umfasst, der darin besteht, das zweite Frequenzband des Frequenzspektrums jeder ermittelten Vibrations- und/oder akustischen Antwort mit zweiten Frequenzbändern von Frequenzspektren von Vibrations- und/oder akustischen Referenzantworten zu vergleichen.

6. Verfahren nach einem der Ansprüche 1 bis 5, wobei der Schritt g) einen Schritt umfasst, der darin besteht, mindestens ein repräsentatives Merkmal des zweiten Frequenzbands des Frequenzspektrums jeder ermittelten Vibrations- und/oder akustischen Antwort zu berechnen.

7. Verfahren nach den Ansprüchen 5 und 6, wobei der Schritt g) einen Schritt umfasst, der darin besteht, die berechneten repräsentativen Merkmale mit entsprechenden Werten der zweiten Frequenzbändern der Frequenzspektren der Vibrations- und/oder akustischen Referenzantworten zu vergleichen.

8. Verfahren nach Anspruch 6, wobei der Schritt h) darin besteht, jedes Kriterium, das sich auf jede erregte Zone des Holzstücks bezieht, ausgehend von dem mindestens einen entsprechenden repräsentativen Merkmal, das in Schritt g) berechnet wurde, und von Rechentafeln zu bestimmen.

9. Verfahren nach einem der Ansprüche 1 bis 8, wobei der Schritt d) einen Schritt umfasst, der darin besteht, die Resonanzfrequenzen mehrerer Resonanzmodi zu messen, die zum ersten analysierten Frequenzband gehören.

10. Verfahren nach einem der Ansprüche 1 bis 9, wobei der Schritt e) und/oder der Schritt f) einen Schritt umfasst, der darin besteht, die Auswirkung jedes Kriteriums bei der Bestimmung der mindestens einen mechanischen Eigenschaft der Faser und/oder des Reißmoduls in Abhängigkeit von der Lokalisierung der entsprechenden erregten Zone zu wichten.

11. Verfahren nach einem der Ansprüche 1 bis 10, wobei der Schritt a) darin besteht, das Holzstück mit Hilfe einer Schlagvorrichtung zu schlagen.

12. Vorrichtung zur zerstörungsfreien Bestimmung (2) des Reißmoduls eines Holzstücks (3), die umfasst:

- Erregungsmittel (7), die ausgebildet sind, um mindestens eine vorbestimmte Zone des Holzstücks derart zu erregen, dass mechanische Vibrationen in dem Holzstück erzeugt werden,
- Detektionsmittel (8), die ausgebildet sind, um eine Vibrations- und/oder akustische Antwort des Holzstücks in Beantwortung auf die Erregung jeder vorbestimmten Zone zu ermitteln,
- Messmittel (9, 11, 12), die ausgebildet sind, um die Abmessungen und die Masse des Holzstücks zu messen, und Bestimmungsmittel (14), die ausgebildet sind, um die Dichte des Holzstücks zu bestimmen,
- erste Analysemittel (17), die ausgebildet sind, um ein erstes Frequenzband (16a) des Frequenzspektrums (16) mindestens einer ermittelten Vibrations- und/oder akustischen Antwort zu analysieren,
- erste Bestimmungsmittel (19), die ausgebildet sind, um mindestens eine mechanische Eigenschaft der Faser des Holzstücks ausgehend von mindestens der Dichte des Holzstücks und der von den ersten Analysemitteln durchgeführten Analyse zu bestimmen, und
- zweite Bestimmungsmittel (22), die ausgebildet sind, um das Reißmodul des Holzstücks ausgehend von mindestens einer mechanischen Eigenschaft der Faser des Holzstücks zu bestimmen, die von den ersten Bestimmungsmitteln bestimmt wurde, **dadurch gekennzeichnet dass** sie ferner umfasst:

- zweite Analysemittel (18), die ausgebildet sind, um ein zweites Frequenzband (16b) mit höheren Frequenzen als das erste Frequenzband des Frequenzspektrums (16) jeder ermittelten Vibrations- und/oder akustischen Antwort zu analysieren,

- dritte Bestimmungsmittel (21), die ausgebildet sind, um für jede erregte Zone ($Z_N$) des Holzstücks ein Kriterium ($C_N$), das sich auf die Anwesenheit und das Ausmaß eines eventuellen Fehlers in der Zone bezieht, ausgehend von der von den zweiten Analysemitteln (18) durchgeführten Analyse zu bestimmen,

wobei die ersten und/oder zweiten Bestimmungsmittel (19, 22) ferner ausgebildet sind, um das Reißmodul und/oder die mindestens eine mechanische Eigenschaft der Faser des Holzstücks, von der ausgehend das Reißmoduls bestimmt wird, in Abhängigkeit von dem oder den von den dritten Bestimmungsmitteln (21) bestimmten Kriterium/Kriterien zu bestimmen.

**13.** Bestimmungsvorrichtung nach Anspruch 12, wobei die Erregungsmittel (7) ausgebildet sind, um mindestens eine vorbestimmte Zone des Holzstücks quer zu erregen.

**14.** Bestimmungsvorrichtung nach Anspruch 12 oder 13, wobei die Verlagerungsmittel (5) ausgebildet sind, um das Holzstück relativ zu den Erregungs- und Detektionsmitteln längs zu verlagern.

**15.** Bestimmungsvorrichtung nach Anspruch 14, wobei die Verlagerungsmittel Transportmittel aufweisen, die ausgebildet sind, um das Holzstück relativ zu den Erregungs- und Detektionsmitteln (7, 8) längs zu transportieren.

**Claims**

**1.** A nondestructive method for determining the modulus of rupture of a piece of wood (3), comprising the following steps:

a) exciting at least one predetermined zone of the piece of wood so as to generate mechanical vibrations in the piece of wood;
b) detecting a vibratory and/or acoustic response of the piece of wood in response to the excitation of each predetermined zone,
c) measuring the dimensions and the mass of the piece of wood so as to determine its density,
d) analyzing a first frequency band (16a) of the frequency spectrum (16) of at least one detected vibratory and/or acoustic response,
e) determining at least one mechanical property of the fiber of the piece of wood from at least the density of the piece of wood determined in step c) and the analysis done in step d),
f) determining the modulus of rupture (MoR) of said piece of wood from at least one mechanical property of the fiber of the piece of wood determined in step e),
**characterized in that** it further comprises the following steps:
g) analyzing a second frequency band (16b), with frequencies higher than the first frequency band, of the frequency spectrum (16) of each detected vibratory and/or acoustic response,
h) determining, for each excited zone ($Z_N$) of the piece of wood, a criterion ($C_N$) relative to the presence and significance of any defect in said zone from the analysis done in step g),

the modulus of rupture and/or at least one mechanical property of the fiber of the piece of wood from which the modulus of rupture is determined being further determined as a function of the criterion or criteria determined step h).

**2.** The method according to claim 1, wherein step a) consists of transversely exciting at least one predetermined zone of the piece of wood.

**3.** The method according to claim 2, wherein step a) consists of transversely exciting several longitudinally offset predetermined zones of the piece of wood.

**4.** The method according to claim 3, which comprises a step consisting of longitudinally moving the piece of wood between each excitation step of the predetermined zone of the piece of wood.

**5.** The method according to one of claims 1 to 4, wherein step g) comprises a step consisting of comparing the second frequency band of the frequency spectrum of each detected vibratory and/or acoustic response with second frequency bands of reference vibratory and/or acoustic response frequency spectrums.

**6.** The method according to one of claims 1 to 5, wherein step g) comprises a step consisting of calculating at least one characteristic that is representative of the second frequency band of the frequency spectrum of each detected vibratory and/or acoustic response.

7. The method according to claims 5 and 6, wherein step g) comprises a step consisting of comparing said calculated representative characteristics to the corresponding values of the second frequency bands of the frequency spectrums of the reference vibratory and/or acoustic responses.

8. The method according to claim 6, wherein step h) consists of determining each criterion relative to each excited zone of the piece of wood from the at least one corresponding representative characteristic calculated in step g) and calculation abacuses.

9. The method according to one of claims 1 to 8, wherein step d) comprises a step consisting of measuring the resonance frequencies of several resonance modes belonging to the first analyzed frequency band.

10. The method according to one of claims 1 to 9, wherein step e) and/or step f) comprises a step consisting of weighting the impact of each criterion in the determination of the at least one mechanical property of the fiber and/or the modulus of rupture as a function of the location of the corresponding excited zone.

11. The method according to one of claims 1 to 10, wherein step a) consists of impacting the piece of wood using an impacting device.

12. A device (2) for the nondestructive determination of the modulus of rupture of a piece of wood (3), comprising:

- excitation means (7) arranged to excite at least one predetermined zone of the piece of wood so as to generate mechanical vibrations in the piece of wood,
- detection means (8) arranged to detect a vibratory and/or acoustic response of the piece of wood in response to the excitation of each predetermined zone,
- measuring means (9, 11, 12) arranged to measure the dimensions and the mass of the piece of wood, and determination means (14) arranged to determine the density of the piece of wood,
- first analysis means (17) arranged to analyze a first frequency band (16a) of the frequency spectrum (16) of at least one detected vibratory and/or acoustic response,
- first determination means (19) arranged to determine at least one mechanical property of the fiber of the piece of wood from at least the density of the piece of wood and the analysis done by the first analysis means, and
- second determination means (22) arranged to determine the modulus of rupture of said piece of wood from at least one mechanical property of the fiber of the piece of wood determined by the first determination means,

**characterized in that** it further comprises:
- second analysis means (18) arranged to analyze a second frequency band (16b), with frequencies higher than the first frequency band, of the frequency spectrum (16) of each detected vibratory and/or acoustic response,
- third determination means (21) arranged to determine, for each excited zone ($Z_N$) of the piece of wood, a criterion ($C_N$) relative to the presence and significance of any defect in said zone from the analysis done by the second analysis means (18),

the first and/or second determination means (19, 22) further being arranged to determine the modulus of rupture and/or the at least one mechanical property of the fiber of the piece of wood from which the modulus of rupture is determined as a function of the criterion or criteria determined by the third determination means (21).

13. The determination device according to claim 12, wherein the excitation means (7) are arranged to transversely excite at least one predetermined zone of the piece of wood.

14. The determination device according to claim 12 or 13, which further comprises movement means (5) arranged to move the piece of wood longitudinally relative to the excitation and detection means.

15. The determination device according to claim 14, wherein the movement means include conveying means arranged to convey the piece of wood along the excitation and detection means (7, 8).

Fig. 1

Fig. 2

Fig. 3

Fig. 4

Fig. 5

Fig. 6

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1950561 A
- CA 2497199
- WO 2004106918 A
- WO 8810415 A